(19) Europäisches Patentamt
European Patent Office
Office européen des brevets

(11) Numéro de publication : **0 036 812**
**B1**

(12) # FASCICULE DE BREVET EUROPÉEN

(45) Date de publication du fascicule du brevet : **30.01.85**

(51) Int. Cl.[4] : **C 07 D501/36**, A 61 K 31/545

(21) Numéro de dépôt : **81400430.5**

(22) Date de dépôt : **20.03.81**

(54) **Nouveaux dérivés des céphalosporines, leur procédé de préparation et les médicaments utilisables comme antibiotiques qui contiennent lesdits dérivés.**

(30) Priorité : **26.03.80 FR 8006757**

(43) Date de publication de la demande : **30.09.81 Bulletin 81/39**

(45) Mention de la délivrance du brevet : **30.01.85 Bulletin 85/05**

(84) Etats contractants désignés : **AT BE CH DE FR GB IT LI LU NL SE**

(56) Documents cités : **FR-A- 2 345 153**
**Le dossier contient des informations techniques présentées postérieurement au dépôt de la demande et ne figurant pas dans le présent fascicule.**

(73) Titulaire : **SANOFI, société anonyme**
**40, Avenue George V**
**F-75008 Paris (FR)**

(72) Inventeur : **Labeeuw, Bernard**
**49, Lotissement des Genêts**
**avenue des Moulins F-34000 Montpellier (FR)**
Inventeur : **Salhi, Ali**
**Résidence Le Belvédère A1 rue Marius Carrieu**
**F-34100 Montpellier (FR)**

(74) Mandataire : **Combe, André et al**
**CABINET BEAU DE LOMENIE 55 rue d'Amsterdam**
**F-75008 Paris (FR)**

**EP 0 036 812 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention concerne des dérivés de la famille des céphalosporines, leur procédé de préparation et leur application en thérapeutique.

Les composés selon l'invention répondent à la formule :

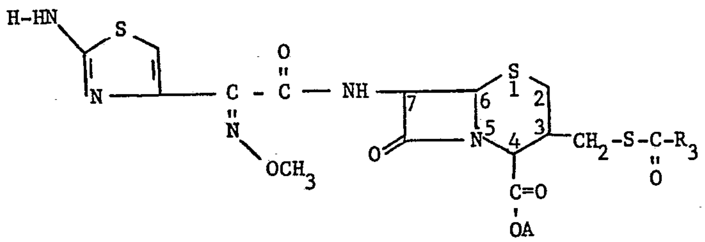

(I)

dans laquelle :

— le groupe

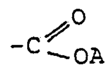

en position 4 est un radical acide, ou un sel alcalin ou alcalino-terreux ou un sel d'amine, ou un radical ester facilement hydrolysable ou métaboliquement labile et pharmaceutiquement acceptable ; et

— $R_3$ est un radical parmi les radicaux

furyle

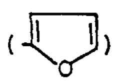

et thiényle

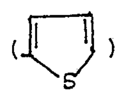

Ledit sel d'amine peut être avantageusement un sel avec la triéthylamine ou avec une éthanolamine.

Par suite de la présence dans la formule d'un groupement oxime, les composés (I) existent sous deux formes isomères syn et anti. Les isomères syn dont l'activité thérapeutique est supérieure sont les composés préférés.

Il est entendu que les composés (I) indiqués ci-dessus peuvent exister :

— soit sous la forme indiquée dans la formule (I),

— soit sous la forme tautomère (I') :

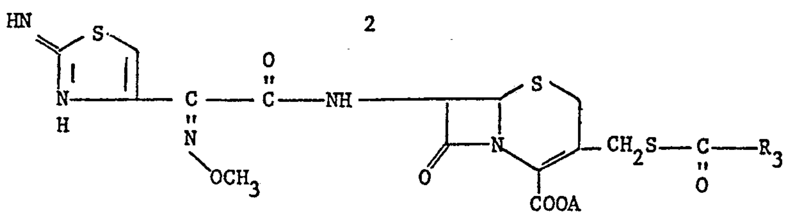

(I')

dans laquelle A et $R_3$ ont les significations indiquées précédemment.

L'art antérieur le plus proche semble être constitué par le brevet français 2 345 153 ; par rapport aux produits décrits dans ce brevet français, les produits de la présente demande se distinguent de façon non prévisible par une activité considérablement améliorée.

L'invention concerne également un procédé de préparation des composés de formule (I) qui comprend l'acylation d'une amino-7 céphalosporine de formule :

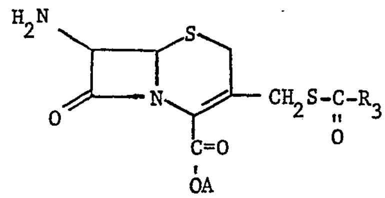

(II)

par l'acide :

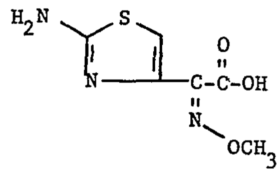

(III)

Avant d'effectuer la réaction d'acylation, il est souhaitable de substituer le groupe amino de l'acide par un groupe protecteur facile à éliminer ultérieurement. On peut utiliser les groupes habituellement utilisés en synthèse organique pour la protection des groupes aminés et en particulier le groupe trityle.

Pour effectuer la réaction d'acylation, il est nécessaire de procéder à l'activation du groupe carboxyle du composé (III) de préférence par transformation en anhydride à l'aide d'un carbodiimide, en général le dicyclohexylcarbodiimide.

La réaction d'activation est effectuée au sein d'un solvant organique convenable tel que le tétrahydrofuranne à une température comprise entre 0 et 50 °C et de préférence à température ambiante. La réaction d'activation est éventuellement facilitée par addition d'un dérivé hydroxylé tel que l'hydroxy-1 benzotriazole.

La solution du réactif d'acylation ainsi obtenue, débarrassée par filtration de la dicyclohexylurée formée, est ajoutée à une solution du composé (II) dans un solvant tel que le tétrahydrofuranne aqueux et en présence d'un agent alcalin tel que la triéthylamine. L'addition des deux réactifs peut aussi s'effectuer dans l'ordre inverse.

Après la réaction d'acylation, le groupe protecteur est éliminé par un procédé connu, en particulier par hydrolyse en milieu acide en utilisant un acide organique tel que l'acide formique ou l'acide trifluoroacétique.

En ce qui concerne les matières premières de la réaction, le composé (III) et ses dérivés dans lesquels le groupe aminé est bloqué par un groupe protecteur sont connus.

Les produits de formule (II) sont connus ou peuvent être préparés selon un procédé connu par action d'un thioacide sur l'acide amino-7 céphalosporanique :

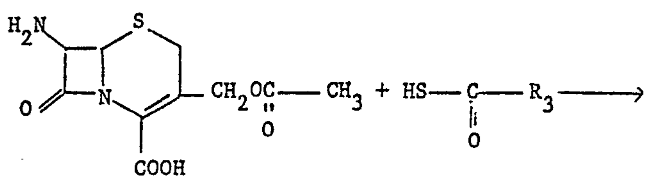

(II)

Les thioacides dont certains sont connus peuvent être obtenus par action d'un sulfhydrate alcalin sur le chlorure ou un anhydride mixte de l'acide correspondant selon la méthode décrite dans Journal of Antibiotics *27,* 573-8, (1974) ou encore par action d'hydrogène sulfuré au sein de la pyridine ou de la triéthylamine sur le chlorure ou un anhydride mixte de l'acide correspondant.

Selon une variante du procédé, on peut obtenir les composés (I) à partir de l'acide amino-7 céphalosporanique et de l'acide (III) qui conduisent au composé (IV). Par action sur (IV) du thioacide

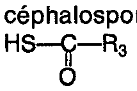

on obtient les composés (I) selon le schéma :

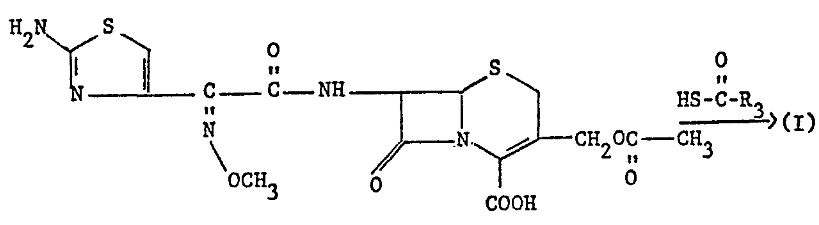

(IV)

Les composés (I) de l'invention dans lesquels A est autre que H s'obtiennent à partir des composés (I) dans lesquels A est H par des réactions connues en elles-mêmes.

Ainsi les sels minéraux sont obtenus par action sur les composés (I) dans lesquels A est H d'une base minérale telle que la soude ou la potasse ou le bicarbonate de sodium en quantité équimoléculaire ; la réaction de salification est réalisée dans un solvant tel que l'eau ou l'éthanol et le sel obtenu est isolé par évaporation de la solution.

Les sels de bases organiques sont obtenus par action, sur une solution de l'acide (I, A = H) dans un solvant ou un mélange de solvants convenable, d'une quantité équimoléculaire de la base organique. Le sel est isolé par précipitation avec l'éther.

Les esters sont obtenus par les procédés connus d'estérification ; par exemple on utilisera avantageusement l'action d'un dérivé halogéné sur un sel tel que le sel de sodium de l'acide ; on réalisera de préférence la réaction dans un solvant capable de dissoudre le dérivé acide de départ par exemple dans le diméthylformamide.

Les isomères de forme syn et anti s'obtiennent par un choix convenable des réactifs.

Les exemples suivants permettent de mieux comprendre la portée de l'invention.

Ainsi qu'il est habituel dans cette famille de composés, les produits suivant l'invention ne présentent pas de point de fusion net mais seulement des points de décomposition ne permettant pas de les caractériser.

Les produits seront donc caractérisés par leur spectre de résonance magnétique nucléaire enregistré à 60 MHz, l'étalon interne étant l'hexaméthyldisiloxane.

Les abréviations suivantes seront utilisées :

— S : singulet
— D : doublet
— D de D : doublet de doublet
— S. el : singulet élargi
— AB : système AB
— J : représente la constante de couplage.

De plus les microanalyses élémentaires ont été effectuées dans chaque cas et sont en accord avec les formules indiquées.

Exemple 1

Acide [(amino-2 thiazolyl-4)-2 méthoxyimino-2 acétamido]-7 (furyl-2 carbonylthiométhyl)-3 céphème-3 carboxylique-4 isomère syn. (CM 31916).

$R_3$= 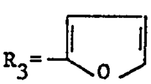 ; A=H

a) Acide [(tritylamino-2 thiazolyl-4)-2 méthoxy-imino-2 acétamido]-7 (furyl-2 carbonylthiométhyl)-3 céphème-3 carboxylique-4 isomère syn.

A une suspension de 44,3 g d'acide (tritylamino-2 thiazolyl-4)-2 méthoxyimino-2 acétique isomère syn dans 400 ml de tétrahydrofuranne anhydre, on ajoute 20,6 g de dicyclohexylcarbodiimide et 13,5 g d'hydroxy-1 benzotriazole. On agite pendant 45 minutes à température ambiante puis on filtre la dicyclohexylurée formée.

On ajoute sous agitation et atmosphère d'azote pendant 45 minutes la solution ainsi préparée à une solution de 28 g d'acide amino-7 (furyl-2 carbonylthiométhyl)-3 céphème-3 carboxylique-4 et 12 ml de triéthylamine dans 140 ml d'eau glacée et 140 ml de tétrahydrofuranne. Pendant toute la durée de l'addition et 30 minutes après la fin de celle-ci, on maintient la température à environ 5 °C par un bain de glace et on maintient le pH à 8,9 par addition d'une solution à 30 % (volume/volume) de triéthylamine dans le mélange tétrahydrofuranne-eau (50-50).

On laisse ensuite sous agitation à température ambiante pendant 20 heures. On évapore le tétrahydrofuranne sous vide et extrait le résidu d'abord 350 ml de chlorure de méthylène, puis une seconde fois avec 150 ml du même solvant. On réunit les extraits organiques, ajoute 100 ml d'eau et acidifie à pH 2 par addition d'une solution d'acide chlorhydrique 6 N. On décante la phase aqueuse, lave la solution organique avec 200 ml d'eau puis sèche sur sulfate de magnésium. On essore le solide qu'on rince avec de l'éther, le produit obtenu est purifié par dissolution dans 150 ml de chlorure de méthylène et reprécipitation par 400 ml d'éther.

On obtient finalement 39,8 g du produit attendu. Par addition de plus d'éther on isole un second jet (3,2 g).

Spectre de RMN (en solution dans le diméthylsulfoxyde) :

1 H à 9,55 ppm (NH CO, D, J = 8 Hz) — 1 H à 8,90 ppm (NH Trit. S. el) — 1 H à 8,0 ppm ($H_5$ furanne, S) — 16 H à 7,25 ppm (H phényle + $H_3$ furanne) — 2 H à 6,70 ppm ($H_5$ thiazole + $H_4$ furanne) — 1 H à 5,65 ppm ($H_7$, D de D, $J_1$ = 8 Hz $J_2$ = 5 Hz) — 1 H à 5,09 ppm ($H_6$, D, J = 5 Hz) — 2 H à 4,15 ppm ($CH_2$-S, AB, $J_{AB}$ = 14 Hz) — 3 H à 3,78 ppm ($CH_3$ ON, S) — 2 H à 3,47 ppm ($CH_2$ en 2, AB, $J_{AB}$ = 16 Hz).

b) CM 31916

A 10 ml d'une solution aqueuse d'acide formique à 50 % (volume/volume) chauffée à 57 °C, on ajoute 3 g du produit tritylé obtenu précédemment. On agite pendant 20 minutes en maintenant la température à 57 °C puis refroidit à température ambiante. On filtre le triphénylcarbinol formé et on le lave avec 5 ml de solution aqueuse d'acide formique à 30 % (volume/volume).

Au filtrat on ajoute 20 ml d'éthanol absolu et on évapore à siccité sous vide. On reprend le résidu dans 10 ml d'éthanol absolu et essore le solide qu'on lave avec de l'éther.

Le solide est redissous dans un mélange de 100 ml d'éthanol absolu et 50 ml d'acétone puis la solution est concentrée à 10 ml. On essore le précipité qu'on lave avec de l'éthanol absolu puis avec de l'éther. Après séchage on obtient 1 g du produit attendu.

Spectre de RMN (en solution dans le diméthylsulfoxyde) :

1 H à 9,50 ppm (NH-CO, D, J = 8 Hz) — 1 H à 8,0 ppm ($H_5$ furanne, S) — 1 H à 7,37 ppm ($H_3$ furanne, D, J = 3 Hz) — 2 H à 7,15 ppm ($NH_2$, S. el) — 2 H à 6,70 ppm ($H_4$ furanne et $H_5$ thiazole) — 1 H à 5,70 ppm ($H_7$, D de D, $J_2$ = 8 Hz $J_2$ = 5 Hz) — 1 H à 5,10 ppm ($H_6$, D, J = 5 Hz) — 2 H à 4,05 ppm ($CH_2$S, AB, $J_{AB}$ = 14 Hz) — 3 H à 3,75 ppm ($CH_3$ON, S) — 2 H à 3,50 ppm ($CH_2$ en 2, AB, $J_{AB}$ = 16 Hz).

En remplaçant l'acide amino-7 (furyl-2 carbonylthiométhyl)-3 céphème-3 carboxylique-4 par l'acide correspondant dans lequel le radical furyle a été remplacé par un radical thiényle on obtient le produit CM 39917 dans lequel :

$R_3$ est 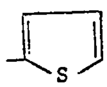

et A est H.

Spectre de RMN (en solution dans le diméthylsulfoxyde) :

1 H à 9,50 ppm (NH-CO, D, J = 8 Hz) — 2 H à 7,92 ppm ($H_3$ thiophène et $H_5$ thiophène) — 3 H à 7,20 ppm ($NH_2$ et $H_4$ thiophène) — 1 H à 6,70 ppm ($H_5$ thiazole, S) — 1 H à 5,70 ppm ($H_7$, D de D, $J_1$ = 8 Hz, $J_2$ = 5 Hz) — 1 H à 5,08 ppm ($H_6$, D, J = 5 Hz) — 2 H à 4,14 ppm ($CH_2$-S), AB, $J_{AB}$ = 14 Hz) — 3 H à 3,77 ppm ($CH_3$ON, S) — 2 H à 3,51 ppm, $CH_2$ en 2, AB, $J_{AB}$ = 16 Hz).

## Exemple 2

Sel de triéthanolamine de l'acide [(amino-2 thiazolyl-4)-2 méthoxyimino-2 acétamido]-7 (thiényl-2 carbonylthiométhyl)-3 céphème-3 carboxylique-4 isomère syn.

On dissout 0,5 g du composé acide CM 31917 dans le mélange de 20 ml d'éthanol absolu et 20 ml d'acétone, puis on ajoute 0,13 g de triéthanolamine et concentre sous vide à 5 ml. On ajoute 20 ml d'éther et essore le précipité qu'on lave avec de l'éther. Après séchage sous vide, on obtient 0,6 g du sel attendu.

Spectre de RMN (en solution dans le diméthylsulfoxyde) :

1 H à 9,60 ppm (NHCO, D, J = 8 Hz) — 2 H à 8,0 ppm ($H_3$ et $H_5$ thiophène) — 3 H à 7,25 ppm ($NH_2$ et $H_4$ thiophène) — 1 H à 6,75 ppm ($H_5$ thiazole, S) — 1 H à 5,70 ppm ($H_7$, D de D, $J_1$ = 8 Hz $J_2$ = 5 Hz) — 1 H à 5,02 ppm ($H_6$, D, J = 5 Hz) — 2 H à 4,12 ppm ($CH_2$S, M) — 3 H à 3,82 ppm ($CH_3$ON, S) — 6 H à 3,67 ppm ($CH_2$OH, M) — 2 H à 3,37 ppm ($CH_2$ en 2, AB, $J_{AB}$ = 16 Hz) — 6 H à 3,04 ppm

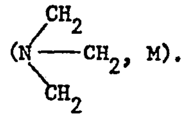

Les produits de l'invention ont été étudiés en ce qui concerne leurs propriétés pharmacologiques et plus spécialement l'action bactériostatique.

L'action bactériostatique in vitro a été déterminée en milieu solide par la méthode des dilutions. L'étude a porté sur des souches Gram$^+$ et Gram$^-$ dont plusieurs connues pour produire des β lactamases.

Les résultats sont exprimés en les concentrations minimales inhibitrices (CMI-µg/ml).

L'efficacité thérapeutique des produits est déterminée dans le modèle septicémique de la souris.

La septicémie est provoquée par inoculation intrapéritonéale de 0,5 ml d'une dilution appropriée de suspension de la souche E. coli Sol RL 90 productrice de céphalosporinase.

Les produits sont administrés en solution dans un tampon phosphate pH 7,0 sous un volume de 0,2 ml par voie sous-cutanée et à raison de 5 doses (mg de produit par kg de poids vif de souris) à des lots de 10 souris 1 et 5 heures après l'inoculation du germe.

Au bout de 4 jours d'observation au cours desquels la mortalité est notée, les doses efficaces 50 % (DE 50) sont calculées par la méthode de Muench et Reed.

Les résultats obtenus figurent dans le tableau I ci-après. Ces résultats montrent clairement la grande activité des produits tant sur les bactéries non productrices de β lactamases que sur les souches productrices.

Par ailleurs les essais effectués jusqu'à ce jour sur les animaux n'ont pas mis en évidence de toxicité particulière pour les produits selon l'invention.

Les produits de l'invention peuvent donc être utilisés comme antibiotiques en médecine humaine ou vétérinaire. Ils présentent un large spectre et peuvent être utilisés dans toutes les infections bactériennes à germes sensibles.

Les produits peuvent être administrés par voie orale, rectale, injectable ou locale.

Les compositions pharmaceutiques sont réalisées à partir des composés (I) sous leur forme acide ou, lorsque leur solubilité est insuffisante, sous forme d'un sel. Ainsi le sel de sodium du composé CM 31916 présente une solubilité supérieure à 20 % dans une solution tampon ayant un pH de 7.

Les compositions pharmaceutiques peuvent être solides ou liquides et se présenter par exemple sous forme de comprimés, gélules, granulés, suppositoires, pommades, crèmes, gels ou préparation injectables.

La posologie peut varier dans de larges proportions en particulier suivant le type et la gravité de l'injecton à traiter et suivant le mode d'administration. Le plus souvent, chez l'adulte par voie injectable, on utilise un produit contenant entre 0,250 g et 4 g de principe actif.

A titre d'exemple de composition pharmaceutique, on peut préparer des ampoules contenant :

| | |
|---|---|
| CM 31916 sel de sodium | 1,10 g |
| Eau pour préparation injectable | 4 ml |

(Voir Tableau I page 7)

Tableau I

Résultats obtenus : en concentrations minimales inhibitrices (μg/ml)

| Souches / N° de produits | non productrices de lactamases | | | productrices de lactamases | | | Septicémie provoquée chez la souris par Escherichia coli SOL RL 90 DE 50 (mg/kg) |
|---|---|---|---|---|---|---|---|
| | Staphylococcus aureus Smith | Escherichia coli A 223 IP | Klebsiella pneumoniae E 55 | Escherichia coli R 69/2 TEM | Escherichia coli SOL RL 90 | Klebsiella pneumoniae RO 30 | |
| 31 916 | 0,25 | 0,5 | 1 | 0,25 | 2 | 8 | 5,4 |
| 31 917 | 0,25 | 0,5 | 1 | 0,5 | 2 | 8 | 3,6 |

**Revendications** (pour les Etats contractants : BE, CH, DE, FR, GB, IT, LI, LU, NL, SE)

1. Produits chimiques de fomule :

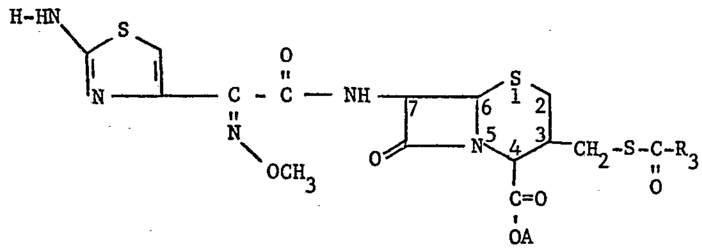

(I)

dans laquelle

— le groupe

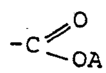

en position 4 est un radical acide, ou un sel alcalin ou alcalino-terreux ou un sel d'amine, ou un radical ester facilement hydrolysable ou métaboliquement labile et pharmaceutiquement acceptable ; et

— $R_3$ représente un radical choisi parmi les radicaux furyle

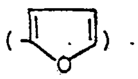

et thiényle

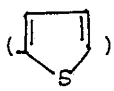

2. Produits selon la revendication 1, caractérisés en ce qu'ils sont sous forme syn ou anti ou en mélange.

3. Produits selon l'une des revendications 1 et 2, caractérisés en ce que le sel d'amine est un sel avec la triéthylamine ou un sel avec une éthanolamine.

4. Procédé de préparation des produits selon l'une des revendications 1, 2 et 3, caractérisé en ce qu'on réalise l'acylation d'une amino-7 céphalosporine de formule :

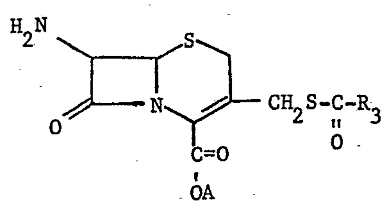

(II)

dans laquelle $R_3$ et A ont la signification donnée dans la revendication 1, par l'acide :

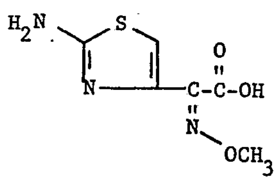

(III)

dans lequel le groupe carbonyle a été activé, ladite acylation étant effectuée dans un solvant en présence d'un agent alcalin.

5. Procédé selon la revendication 4, caractérisé en ce que ladite activation du groupe carbonyle a été réalisée par transformation dudit groupe en anhydride.

6. Procédé selon l'une des revendications 4 et 5, caractérisé en ce que ledit agent alcalin est la triéthylamine.

7. Procédé selon l'une des revendications 4, 5 et 6, caractérisé en ce que, avant d'effectuer la réaction d'acylation, le groupe amino de l'acide est substitué par un groupement protecteur qui pourra être ultérieurement éliminé.

8. Procédé selon la revendication 7, caractérisé en ce que ledit groupement protecteur est le groupe trityle.

9. Procédé selon la revendication 4, caractérisé en ce que l'activation du groupe carbonyle de l'acide est effectuée par transformation de ce groupe en anhydride à l'aide d'un carbodiimide.

10. Médicaments utilisables comme antibiotiques, en médecine humaine et vétérinaire, caractérisés en ce qu'ils comportent, comme substance active, au moins un produit selon l'une des revendications 1 à 3.

11. Médicaments selon la revendication 10, caractérisés en ce qu'ils sont conditionnés pour être administrés par voie orale, rectale, injectable ou locale.

12. Médicaments selon la revendication 10, caractérisés en ce qu'ils sont conditionnés pour être administrés par voie injectable et qu'ils contiennent de 0,250 à 4 g de produit selon l'une des revendications 1 à 3.

**Revendications** (pour l'Etat contractant AT)

1. Procédé de préparation de nouveaux dérivés des céphalosporines de formule générale :

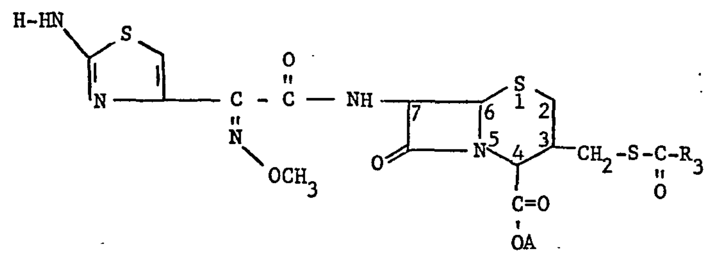

(I)

dans laquelle :

— le groupe

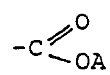

en positon 4 est un radical acide, ou un sel alcalin ou alcalino-terreux ou un sel d'amine, ou un radical ester facilement hydrolysable ou métaboliquement labile et pharmaceutiquement acceptable ; et

— $R_3$ représente un radical choisi parmi les radicaux furyle

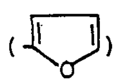

et thiényle

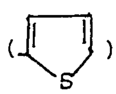

caractérisé en ce que l'on réalise l'acylation d'une amino-7 céphalosporine de formule :

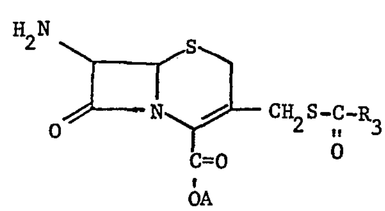

(II)

dans laquelle $R_3$ et A ont la signification donnée ci-dessus, par l'acide :

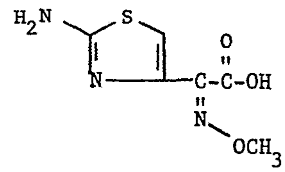

(III)

dans lequel le groupe carbonyle a été activé, ladite acylation étant effectuée dans un solvant en présence d'un agent alcalin.

2. Procédé selon la revendication 1, caractérisé en ce que ladite activation du groupe carbonyle a été réalisée par transformation dudit groupe en anhydride.

3. Procédé selon l'une des revendications 1 et 2, caractérisé en ce que ledit agent alcalin est la triéthylamine.

4. Procédé selon l'une des revendications 1, 2 et 3, caractérisé en ce que, avant d'effectuer la réaction d'acylation, le groupe amino de l'acide est substitué par un groupement protecteur qui pourra être ultérieurement éliminé.

5. Procédé selon la revendication 4, caractérisé en ce que ledit groupement protecteur est le groupe trityle.

6. Procédé selon la revendication 4, caractérisé en ce que l'activation du groupe carbonyle de l'acide est effectuée par transformation de ce groupe en anhydride à l'aide d'un carbodiimide.

7. Médicaments utilisables comme antibiotiques, en médecine humaine et vétérinaire, caractérisés en ce qu'ils comportent, comme substance active, au moins un produit préparé selon l'une des revendicatons 1 à 6.

8. Médicaments selon la revendication 7, caractérisés en ce qu'ils sont conditionnés pour être administrés par voie orale, rectale, injectable ou locale.

9. Médicaments selon la revendication 7, caractérisés en ce qu'ils sont conditionnés pour être administrés par voie injectable et qu'ils contiennent de 0,250 à 4 g de produit préparé selon l'une des revendications 1 à 6.

**Claims** (for the Contracting States : BE, CH, DE, FR, GB, IT, LI, LU, NL, SE)

1. Chemical products of formula :

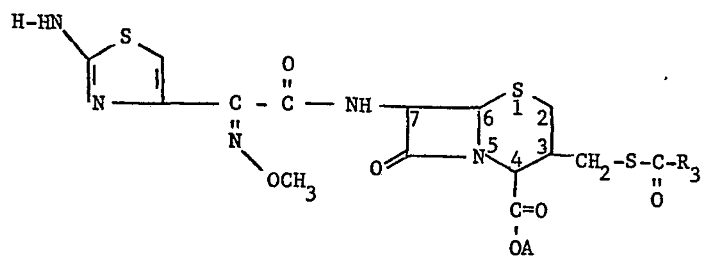

(I)

in which :

— the group

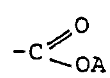

in 4 position in an acid radical, or an alkaline or alkaline-earth salt or an amine salt, or an ester radical easily hydrolysable or metabolically labile and pharmaceutically acceptable,

— and $R_3$ represents a radical chosen from the furyl

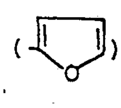

and thienyl

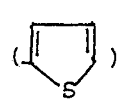

radicals.

2. Products according to claim 1, characterized in that they are in syn or anti form or in mixture.

3. Products according to any one of claims 1 and 2, characterized in that the amine salt is a salt with triethylamine or a salt with ethanolamine.

4. Process for preparing the products according to any one of claims 1, 2 and 3, characterized in that an amino-7 cephalosporin of formula :

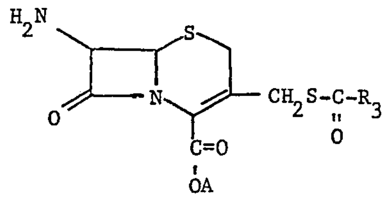

(II)

wherein $R_3$ and A are as defined in claim 1, is acylated by the acid :

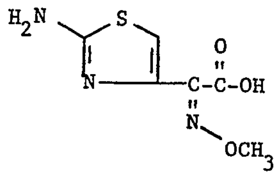

(III)

in which the carbonyl group has been activated, said acylation being effected in a solvent in the presence of an alkaline agent.

5. Process according to claim 4, characterized in that the activation of the carbonyl group is effected by conversion of this group into anhydride.

6. Process according to any one of claims 4 and 5, characterized in that the said alkaline agent is triethylamine.

7. Process according to any one of claims 4, 5 and 6, characterized in that before effecting the acylation reaction, the amino group of the acid is substituted by a protector group which may be subsequently eliminated.

8. Process according to claim 7, characterized in that the said protector group is the trityl group.

9. Process according to claim 4, characterized in that the activation of the carbonyl group is effected by conversion of this group into anhydride with the aid of a carbodiimide.

10. Drugs usable in human and veterinary medicine as antibiotics, characterized in that they comprise, as active substance, at least one product according to any one of claims 1 to 3.

11. Drugs according to claim 10, characterized in that they are prepared for oral, rectal, injectable or local administration.

12. Drugs according to claim 7, characterized in that they are prepared to be administered by the injectable route and contain between 0.250 and 4 g of the product according to any one of claims 1 to 3.

**Claims** (for the Contracting State AT)

1. Process for preparing novel cephalosporin derivatives of general formula :

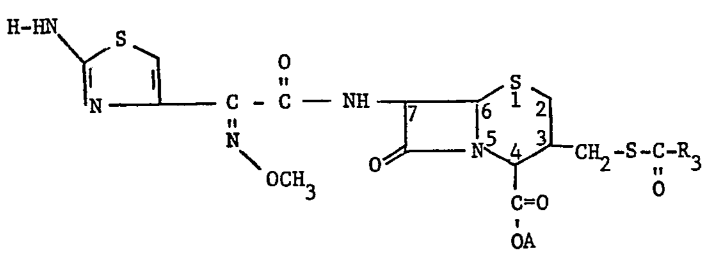

(I)

wherein :

— the group

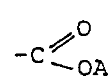

in 4 position is an acid radical, or an alkaline or alkaline-earth salt or an amine salt, or an ester radical easily hydrolysable or metabolically labile and pharmaceutically acceptable,

— and $R_3$ represents a radical chosen from

the furyl

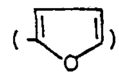

and thienyl

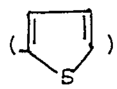

radicals,

characterized in that an amino-7 cephalosporin of formula :

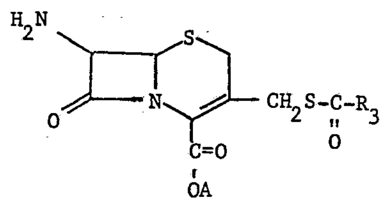

(II)

wherein $R_3$ and

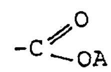

are as defined hereinabove, is acylated by the acid :

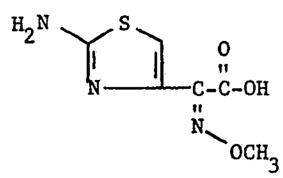

(III)

wherein the carbonyl group can be activated, said acylation being effected in a solvent in the presence of an alkaline agent.

2. Process according to claim 1, characterized in that the activation of the carbonyl group is effected by conversion of this group into anhydride.

3. Process according to any one of claim 1 and 2 and 5, characterized in that as alkaline agent triethylamine is used.

4. Process according to any one of claims 1, 2 and 3, characterized in that before effecting the acylation reaction the amino group of the acid of formula III is substituted by a protector group which may be subsequently eliminated.

5. Process according to claim 4, characterized in that the said protector group is a trityl group.

6. Process according to claim 1, characterized in that the activation of the carbonyl group of the acid of formula (III) is effected by conversion of this group into anhydride with the aid of a carbodiimide.

7. Application of the novel cephalosporin derivatives of formula (I) that can be prepared according to any one of claims 1 to 6, as active agents for the preparation of drugs usable in human and veterinary medicine as antibiotics.

8. Application according to claim 7 on condition that the drugs are prepared for oral, rectal, injectable or local administration.

9. Application according to claim 8, characterized in that the drugs are prepared to be administered by the injectable route and contain between 0.250 and 4 g of the cephalosporin derivatives that can be prepared according to any one of claims 1 to 6.

**Ansprüche** (für die Vertragsstaaten : BE, CH, DE, FR, GB, IT, LI, LU, NL, SE)

1. Chemische Produkte der Formel :

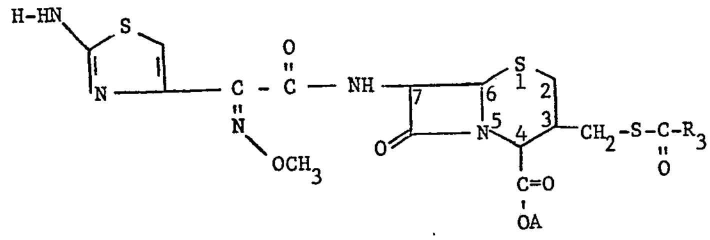

(I)

worin :

die Gruppe

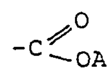

in Position 4 ein Säureradikal oder ein Alkali- oder Erdalkali- oder ein Aminsalz oder ein leicht hydrolysierbarer oder metabolisch labiler und pharmazeutisch akzeptabler Esterrest ist ;

und $R_3$ für ein Radikal, ausgewählt aus

den Furyl-

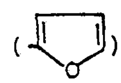

und Thienyl-

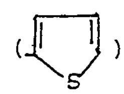

radikalen steht.

2. Produkte nach Anspruch 1, dadurch gekennzeichnet, daß sie in syn- oder anti-Form oder als Mischung dieser Formen vorliegen.

3. Produkte nach einem der Ansprüche 1 und 2, dadurch gekennzeichnet, daß das Aminsalz ein Salz mit Triäthylamin oder ein Salz mit einem Äthanolamin ist.

4. Verfahren zur Herstellung der Produkte nach einem der Ansprüche 1, 2 und 3, dadurch gekennzeichnet, daß man die Acylierung eines 7-Aminocephalosporins der Formel :

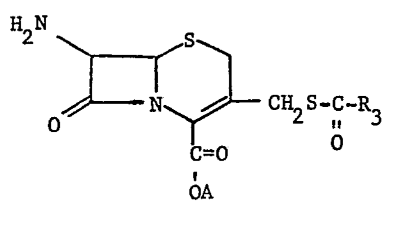

(II)

worin $R_3$ und A die in Anspruch 1 angegebene Bedeutung haben, mit der Säure :

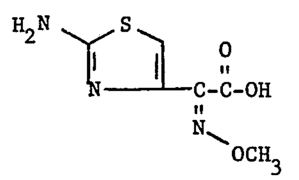

(III)

worin die Carbonylgruppe aktiviert sein kann, durchführt, wobei die Acylierung in einem Lösungsmittel in Gegenwart eines alkalischen Agens vorgenommen wird.

5. Verfahren nach Anspruch 4, dadurch gekennzeichnet, daß die Aktivierung der Carbonylgruppe durch Umwandlung dieser Gruppe in ein Anhydrid bewirkt worden ist.

6. Verfahren nach einem der Ansprüche 4 und 5, dadurch gekennzeichnet, daß als alkalisches Agens Triäthylamin eingesetzt wird.

7. Verfahren nach einem der Ansprüche 4, 5 und 6, dadurch gekennzeichnet, daß die Aminogruppe der Säure vor der Durchführung der Acylierungsreaktion mit einer nachträglich abspaltbaren schutzgruppe substituiert worden ist.

8. Verfahren nach Anspruch 7, dadurch gekennzeichnet, daß die Schutzgruppe eine Tritylgruppe ist.

9. Verfahren nach Anspruch 4, dadurch gekennzeichnet, daß die Aktivierung der Carbonylgruppe der Säure durch Umwandlung dieser Gruppe in ein Anhydrid mittels eines Carbodiimids vorgenommen wird.

10. Medikamente, verwendbar in der Human- und Veterinärmedizin als antibiotika, dadurch gekennzeichnet, daß sie als Wirkstoff wenigstens ein Produkt nach einem der Ansprüche 1 bis 3 enthalten.

11. Medikamente nach Anspruch 10, dadurch gekennzeichnet, daß sie oral, rektal, injizierbar oder lokal verabreichbar formuliert sind.

12. Medikamente nach Anspruch 10, dadurch gekennzeichnet, daß sie zur Verabreichung durch Injektion formuliert sind und 0,250 bis 4 g des Produktes nach einem der Ansprüche 1 bis 3 enthalten.

**Ansprüche** (für den Vertragsstaat AT)

1. Verfahren zur Herstellung neuer Cephalosporinderivate der allgemeinen Formel :

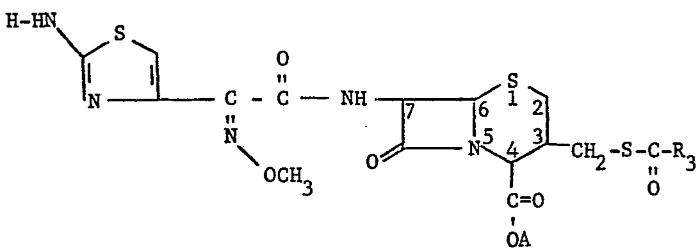

(I)

worin :

die Gruppe

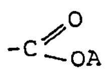

in Position 4 ein Säureradikal oder ein Alkali- oder Erdalkali- oder ein Aminsalz oder ein leicht hydrolysierbarer oder metabolisch labiler und pharmazeutisch akzeptabler Esterrest ist ; und $R_3$ für ein Radikal, ausgewählt aus

den Furyl-

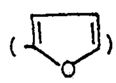

und Thienyl-

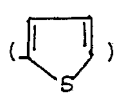

radikalen steht,

dadurch gekennzeichnet, daß man die Acylierung eines 7-Aminocephalosporins der Formel :

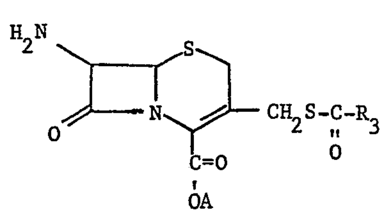

(II)

worin $R_3$ und

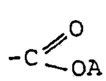

die oben angegebene Bedeutung haben, mit der Säure :

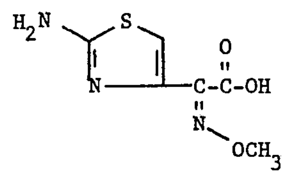

(III)

worin die Carbonylgruppe aktiviert sein kann, durchführt, wobei die Acylierung in einem Lösungsmittel in Gegenwart eines alkalischen Agens vorgenommen wird.

2. Verfahren nach Anspruch 1, dadurch gekennzeichnet, daß die Aktivierung der Carbonylgruppe durch Umwandlung dieser gruppe in ein Anhydrid bewirkt worden ist.

3. Verfahren nach einem der Ansprüche 1 und 2, dadurch gekennzeichnet, daß als alkalisches Agens Triäthylamin eingesetzt wird.

4. Verfahren nach einem der Ansprüche 1, 2 und 3, dadurch gekennzeichnet, daß die Aminogruppe der Säure der Formel (III) vor der Durchführung der Acylierungsreaktion mit einer nachträglich abspaltbaren Schutzgruppe substituiert worden ist.

5. Verfahren nach Anspruch 4, dadurch gekennzeichnet, daß die Schutzgruppe eine Tritylgruppe ist.

6. Verfahren nach Anspruch 1, dadurch gekennzeichnet, daß die Aktivierung der Carbonylgruppe der Säure der Formel (III) durch Umwandlung dieser Gruppe in ein Anhydrid mittels eines Carbodiimids vorgenommen wird.

7. Verwendung der nach einem der Ansprüche 1 bis 6 herstellbaren neuen Cephalosporinderivate der Formel (I) als Wirkstoffe zur Herstellung von Medikamenten, welche in der Human- und Veterinärmedizin als Antibiotika einsetzbar sind.

8. Verwendung nach Anspruch 7 mit der Maßgabe, daß die Medikamente oral, rektal, injizierbar oder lokal verabreichbar formuliert sind.

9. Verwendung nach Anspruch 8, dadurch gekennzeichnet, daß die Medikamente zur Verabreichung durch Injektion formuliert sind und 0,250 bis 4 g der nach einem der Ansprüche 1 bis 6 herstellbaren Cephalosporinderivate enthalten.